# EUROPEAN PATENT SPECIFICATION

(11) **EP 2 424 886 B1**
(45) Date of publication and mention of the grant of the patent: **17.04.2013**
(21) Application number: 10718568.8
(22) Date of filing: 29.04.2010
(51) Int. Cl.: C07K 14/435, C08J 5/24, C12N 9/10, C09J 189/00, C12P 21/00, C08L 89/00

(54) **COMPOSITE MATERIAL COMPRISING CROSSLINKABLE RESIN OF PROTEINOUS MATERIAL**
EIN QUERVERNETZTES HARZ EINES PROTEINÖSEN MATERIALS ENTHALTENDES VERBUNDMATERIAL
MATÉRIAU COMPOSITE COMPRENANT UNE RÉSINE RÉTICULABLE DE MATÉRIAUX PROTÉIQUES

(30) Priority: 29.04.2009 GB 0907323
(43) Date of publication of application: 07.03.2012
(73) Proprietor: Dynea OY, 00530 Helsinki (FI)
(72) Inventor: W.G. VAN HERWIJNEN, Hendrikus, A-3464 Hausleiten-Goldgeben (AT); JOBBER, Andrew, A-3500 Krems (AT); PIETZSCH, Markus, 06114 Halle (Saale) (DE); JACOB, Matthias, 04107 Leipzig (DE)
(74) Representative: Derks, Wilbert
(86) International application number: PCT/EP2010/055865
(87) International publication number: WO 2010/125163

(56) References cited:
- EP-A2- 0 726 317
- WO-A1-03/000060
- WO-A1-2009/077571
- US-A1- 2005 020 506

## Description

### 1. Field of the Invention

The present invention relates to composite material comprising cross-linkable resin based on naturally occurring and/or degradable material.

### 2. Description of the Related Art

Cross-linkable resins are well known, and are often used in applications that require high heat resistance and high hardness. The resins are often used in composite materials where the resin binds particles, fibres or sheets (hereinafter also generally denoted as filler). In contrast to thermoplastic resins, the cross-linked resins do not melt. Such resins can for example be based on polyesters, acrylic resins and/or aminoplast resins. Because of the cross-links, the cured resins generally are difficult to break down in nature. A number of applications for composite materials require these beneficial properties to exist, but at the same time their environmental impact would be minimised by using biologically occurring materials, which preferably are degradable resins, at least when the products made with these composite materials are disposed of.

As naturally occurring, and/or degradable materials, it is known to use for example polylactic acid, polycarbohydrates or proteins. Sometimes these polymers are crosslinked. Proteins can be crosslinked with aldehydes; such as formaldehyde, glutaraldehyde and the like. Such crosslinking generally takes place under heating of the resin system. Other systems are using starch-based material, which can be crosslinked for example by etherification, or reactions with poly-isocyanates.

In view of environmental concerns, there is a need for further development of composite materials with resin systems having high hardness and/or strength.
US 2005/0020506 A1 relates to bio-prosthetic devices and to chemically crosslinked collagen based carriers comprising demineralized bone matrix (DBM) and to the use of these materials as implants such as osteoinductive implants. In particular it relates to slurry composition of collagen protein and demineralized bone matrix that is used as implant for tissue engineering.

### BRIEF SUMMARY OF THE INVENTION

The present invention relates to composite materials comprising more than 50 wt% filler and resin comprising proteinous material that contains proteins or polypeptides that are cross-linkable by enzymes, and enzymes suitable for said crosslinking.

The composite material with crosslinked resin composition is durable under dry as well as under humid or wet conditions. A further advantage is that the energy input to crosslink the resin is substantially lowered in comparison to heat curable resins and that for the crosslinking reaction no hazardous chemicals need to be used.

The present invention furthermore relates to the use of such composite materials for foundry moulds or wood panels, and to a method of making foundry moulds or wood panels by shaping a composite material consisting of resin and fillers; the resin comprising proteinous material that contains proteins or polypeptides that can be crosslinked via enzymatic reaction(s) by using suitably capable enzymes.

The present invention furthermore relates to a process for binding of fillers like fibres, particles or sheets, the binder comprising proteinous material that contains proteins or polypeptides as a cross-linkable material and enzyme as catalyst. The word resin is in such case used interchangeably with the word binder.

The invention furthermore relates to two-component adhesives for fillers like wood or veneer, wherein one component is a proteinous material (containing proteins and/or polypeptides), and the second component is an enzyme, capable of cross-linking the proteins and/or polypeptides in said proteinous material.

### BRIEF DESCRIPTION OF THE DRAWINGS

The features and advantages of the invention will be appreciated upon reference to the following drawings, in which:

Figure 1 is a graphical depiction of an embodiment of the present invention;

Figure 2 is a graphical depiction of an embodiment of the present invention;

Figure 3 is a graphical depiction of an embodiment of the present invention; and

Figure 4 is a graphical depiction of an embodiment of the present invention.

### DESCRIPTION OF ILLUSTRATIVE EMBODIMENTS

The following is a description of certain embodiments of the invention, given by way of example only and with reference to the drawings. Proteinous materials containing proteins or polypeptides that are crosslinked with enzymes are known, but these systems generally are used in the food or feed industry. It was unexpected, that the cross-linkable resin system can actually serve in high strength industrial applications - even in those in which (very) low amount of proteinous material is used (unlike the food and feed industry where compositions contain typically more than 50% protein on dry weight).

With cross-linking it is meant in the present application, the formation of covalent bonds. Van der Waals or ionic interactions are known to cause a certain cohesive force, like in gelatine, but the resin systems of the present invention are moisture resistant and can be substantially harder.

The proteinous material used for the binder may be procured from vegetable (including algal), animal, fungal and/or microbial (bacterial, yeast) sources. The material may, partially, or not be separated from other source substances (e.g. polysaccharides). The proteinous material may also comprise proteins hydrolysed to various degrees to give polypeptides with a controlled molecular weight or to give shorter chains or more linear chains, preferably consisting of at least 10 amino acids.

Furthermore, synthetic polypeptides can be used, like poly-lysine, or poly-amino acids with a relatively large amount of primary amino groups. Depending on the type of enzymatic system, other functional groups than amines may be chosen.

Furthermore, the material may be combined with other crosslinkable polymers or oligomers, like amino functionalized-polyethyleneglycol, amino-functional acrylic resin and akyldiamines. It is preferred to have such compounds present in an amount of less than 40 wt%, preferably less than 20 wt% of the resin.

In this application, % or wt% is defined as the dry weight in the composition or resin as stated.

For certain applications it is desired to have the composition degradable, preferably bio-degradable.

It is preferred to have a resin with proteinous material comprising 0.04 - 8.3 meq reactive groups per gram of resin, preferably 0.1 - 2.0 meq/g reactive groups.

In a preferred embodiment of the invention, the enzyme used for crosslinking is a transglutaminase (EC 2.3.2.13). This enzyme catalyses the acyl rearrangement of a γ-carboxyl group of a glutamine residue and the ε-aminogroup of a lysine residue. It can also utilise primary amine functional groups in addition to the ε-amino groups from lysine. Therefore bi or multifunctional compounds with primary amine groups (from synthetic, agricultural or natural sources) can be used in combination with the proteinous material. In this embodiment, the proteinous material contains lysine and glutamine amino acids. The enzyme transglutaminase is well known in the food industry where it is used to cross-link soft food like fish or meat products (trade name e.g. Activa WM, Ajinomoto Inc., Japan).

The transglutaminase can be also obtained from recombinant-DNA production techniques. For example, transglutaminase from Bacillus, which is Ca2+ independent is a suitable source, and is described in EP07263.

A number of other enzymes can be used for covalently crosslinking proteins. Suitable systems include (i) protein disulphide isomerase (EC 5.3.4.1) which catalyses the reduction of disulphide groups to reactive suphydryl residues, followed by oxidation; (ii) sulphydryl oxidase (EC 1.8.3.2) or thiol oxidase aids in the here above described oxidation reaction; (iii) polyphenol oxidase (EC 1.14.18.1; also known for example as catechol oxidase, tyrosine oxydase, phenol oxidase) catalyses the oxidation of phenols to ortho-diphenols and subsequently to ortho-diquinones; the diquinones react with suphydrylgroups or with amine groups to form protein cross-links; (iv) lysyl oxidase (EC 1.4.3.13) or lysyl-protein-6-oxidase is an enzyme which cross-links collagen or elastin, and it catalyses the oxidative deamination of lysine to a semi-aldehyde, which reacts with other amino acids; and (v) peroxidase (EC 1.11.1.7) causes reactive species through hydroxylation or peroxidation activity to be able to cross-link with other proteins.

In a preferred embodiment of the invention, the enzymes used for crosslinking are adapted to the preferred reaction conditions for the envisioned application. For example, the enzyme may be adapted to be most active at room temperature, or at 50 or 80 °C.

In a further preferred embodiment, the transglutaminase is obtained from genetically modified microorganisms allowing the system to function under required conditions. It is envisaged that selected mutations, can give rise to enzymes that are able to work at room temperature and pressure with higher specific activities; hence a self set system is possible.

The filler in the composite material of the invention can vary greatly. Suitable fillers include fibres, particles or sheets. The fibers, particles or sheets may be of organic nature or inorganic. Suitable fillers are sand, clay, pigments, wood powder, wood fibres, glass fibers, stone fibers, veneer sheets, non-woven paper or felt sheets. In a preferred embodiment, the filler is an inorganic filler and/or wood. Some fillers may contain proteinous material.

The size of filler components may vary. For example, foundry sand having an approximate size of at least about 0.5 mm may be used. In a further embodiment, mineral (glass or stone) wool, with fibres having a diameter size of about at least greater than 0.3 mm and a few centimeters in length may be used.

In one preferred embodiment of the invention, inorganic filler is used. Preferably, the average particle size (measured with sieving) is about 0.1 mm or more, more preferred about 0.3 mm or more. Generally the particle size will be about 5 mm or less, preferably about 1 mm or less.

In a further preferred embodiment, wood fibres are used. Wood fibres used preferably have particle sizes where 70% of the particles are larger than 1.0 mm. Depending on the application, such as production of particle chip board, the particles may be much larger. Sizes of wood fibres or wood chips may be at least to 10 cm, or even up to 20 cm.

Generally, the filler will comprise an amount of protein of about 20 wt% or less, and preferably about 10 wt% or less, and even about 5 wt% or less (relative to the dry weight of the filler). Further, it will be appreciated that according to conventional protein preparation techniques, the resulting product contains less than 10% starch. For example, wood may comprise some proteinous material. Further, one of ordinary skill in the art will appreciate that in the context of the present application wood is not considered a starch. However, the proteinous material of the filler - if any - will not significantly influence the crosslinking of the resin because of the low amount.

The amount of resin comprising the proteinous material in the composite material may vary, and is preferably between 0.01 and 50 wt%. More preferably, the amount of proteinous material is 30% or less, and even more preferably 10 wt% or less. Preferably, the amount of proteinous material is 0.1 wt% or more.

In one embodiment, substantially all the resin is the proteinous material. In another embodiment, more than 50wt% of the resin is the proteinous material, preferably more than 80 wt%.

In one embodiment, substantially all the proteinous material consists of proteins and/or polypeptides. In another embodiment, about 5 wt% of the proteinous material consists of proteins and/or polypeptides, preferably more than 10wt%, and more preferably more than 30 wt%. The relative amount of components in the proteinous material apart from proteins and/or polypeptides is not so important, as that act merely as a diluent (if not reactive). It is important to have crosslinking between the reactive components, with an amount of reactive groups, preferably as defined above.

The enzyme activity is preferably between 0.5 and 50 Units per gram of proteinous material. More preferably, the enzyme activity is between 1 and 25 Units per gram of proteinous material. Hence, the amount of enzyme can be less than 1 wt% or even less than 0.1 wt%. Generally, the enzyme is diluted to a certain standard activity, and it will be understood that if an enzyme composition is used with high activity, less of that composition can be used and visa versa.

The kinetics of the enzymatic crosslinking can be controlled by the activity of the enzyme, the enzyme concentration, temperature, pH, buffer control, ionic strength, and inhibition with a suitable chemical agent. It is also possible to deactivate the enzymatic activity by heat, a pH change or other inhibitors. By this it is possible to produce a material, which has a gradient in mechanical properties such as compression strength, tensile strength, bending strength and shear strength.

The amount of filler may vary, and is between 70 and 99.99 wt%, preferably about 85 wt% or more. The amount most preferably used is about 90 wt% or more. Preferably, the amount of filler is 99.9% or less. In view of the high amount of filler, the crosslinkable resin primarily acts as a binder, but - depending on the amount and type of filler - the resin will not fill all voids between the filler. Also, the resin may be used as a binder or adhesive for sheets of wood.

In one preferred embodiment, the new crosslinkable system is used for foundry moulds. With the current mainstream foundry binders there are a number of health, safety and environmental issues. For example, the known hotbox and shell moulding resins can give rise to significant formaldehyde emissions and odours; whilst furfuryl alcohol based self set resins are coming under increased scrutiny and public debate.

The system according the present invention obviates these problems, because the system can be based on biological materials, which are applied in a way precluding emission of hazardous chemicals.

In the following, several applications are described with the example of foundry moulds. It is however understood by the skilled man, that the technology described can be used with other inorganic or organic fibres, particles or sheet.

The enzyme addition can be made in several ways, including (i) a protein / peptide solution and enzyme (dry or solution) premix is prepared before mixing on sand; (ii) a sand and protein / peptide solution premix is prepared to which the enzyme (dry or in solution) is added with further mixing; (iii) a sand and enzyme (dry or in solution) premix is prepared to which the protein / peptide solution is added with further mixing. Furthermore, sand can be reclaimed by removing the crosslinked protein upon washing with a buffer solution containing a protease.

In a preferred embodiment of the invention, a proteinous solution is used to coat or to partially coat inorganic or organic fibres, particles, or sheets. The proteinous solution is dried, and the product can be stored, shaped or otherwise handled for further use. In a next step, the preferably shaped product is rehydrated, and at the same time or thereafter, enzyme is added, such as to cause crosslinking.

In another preferred embodiment, a freshly prepared proteinous solution containing the enzyme is mixed with inorganic or organic fibres, particles, or sheet, and quickly dried to preclude substantial reaction of the enzyme and protein. The resulting product can be stored, shaped or otherwise handled for further use. In a next step, the preferably shaped product is rehydrated, such as to cause crosslinking.

Following the addition of the enzyme, there will be a period of chain growth. Initially this will allow a "work time" where the filler can be shaped, followed by a "strip time", where there should be no disturbance of the shaped product, allowing the binder to achieve a robust handleable strength.

### a) Proteinous composition plus enzyme addition with dehydratation and rehydratation.

In this embodiment, the proteinous solution with both the proteinous base material, and the cross-linking enzyme is mixed with sand and then quickly dried whilst being mixed, in a method similar to the Novolac coating of sand. The resultant coated sand can then be transported to a foundry and stored for use when required. The coated sand is then rehydrated / solvated with water, or a salt or buffer containing solution, or a mixture of water/solvent (solvents examples include: alcohols, glycerol, esters, and alkoxy esters) or an organic solvent mixture; to such an extent as to permit adhesion and cross-linking reaction when blown or rammed into a mould or corebox and then subjected to a suitable drying or dehydration process. Such a drying process maybe analogous to the Hotbox process or Shell moulding process as already known in the art. Alternatively, vacuum, with or without heat, could result in the hardening of the binder through dehydration. An advantage of this system over the current novolac / hexamine system is that there is no formaldehyde emission.

### b) Rehydration + enzyme followed by crosslinking / and possibly dehydration

This method can be applied as described in the former paragraph, and the proteinous solution (without crosslinking enzyme) is mixed with sand and then dried whilst being mixed. Again, the resultant coated sand can be transported to a foundry and stored for use when required. The coated sand is then rehydrated / solvated with water, a mixture of water/solvent (solvent examples include: alcohols, glycerols, esters and alkoxyesters) or an organic solvent mixture. To this, and with the aid of a foundry sand mixer, a charge of active enzyme is made and mixed in. This charge may be either a dry powder, or an enzyme in solution. This enzyme can for example crosslink specific amino acid functional groups on the protein or peptide chains, resulting in an extended crosslinked protein network.

### c) Enzyme addition followed by crosslinking / and possibly dehydration

In a way analogous to that described above, a low viscosity (<1000mPas) proteinous or peptide solution can be mixed with sand on site at the foundry. To this, an enzyme is added that can specifically crosslink amino acid functional groups on the protein or peptide chains, resulting in an extended crosslinked protein network. The addition of this enzyme can be either as a dry powder or as a solution. However, in this case, the solution is stable (little, or slow enzyme crosslinking reactions) until some characteristic of the solution is changed, to enhance the speed of the enzymatic reaction. Thus, the enzyme may become active on combination with the proteinous / peptide solution by for example changing the pH, adding a co-enzyme, or changing the temperature or ionic strength.

Some modifications to the foundry practice could also be considered; such as a combination of dehydration through the application of heat, and enzyme crosslinking. Practices could be dried in a Hotbox (about 200°C), Warm box (from about 50 to 80°C), and/or can be self setting in a Warm Room (from about 25 to 50°C). A dehumidifier may also aid a Self Set Warm Room process.

The invention will be elucidated with the following non-limiting examples

### Example 1

For the production of compression test specimen, 1000 g of Sand (Quartz sand) of the specification H32 was mixed with 10 g of Sodium-Caseinate. The dry mixture was mixed subsequently with a buffer solution (Tris/HCl, 20 mM, pH 7) containing varying amounts of the enzyme Transglutaminase (TG, Activa WM, Ajnomoto Inc. approximately specific activity 100 U / g). All components were cooled previously to a temperature of 4 °C to prevent cross-linking from occurring to quickly. For each specimen, 163 g of the mixture was filled into a metal mould and was compacted with a ram (3 blows) to form a cylindrical sample body with the dimensions of 50 x 50 mm (height and diameter). The moulds were closed to prevent evaporation and then they were placed in a drying oven for one hour with a temperature of 50 °C.

The mechanical testing was carried out at a material testing device of the company Zwick (Zwick Z020). The sample bodies, still being wet, were mounted on the lower compression plate and then compressed with a speed of 50 mm/min until breakdown. (The upper compression plate was moving downwards with 1 mm/min until a preload of 5 N was reached).

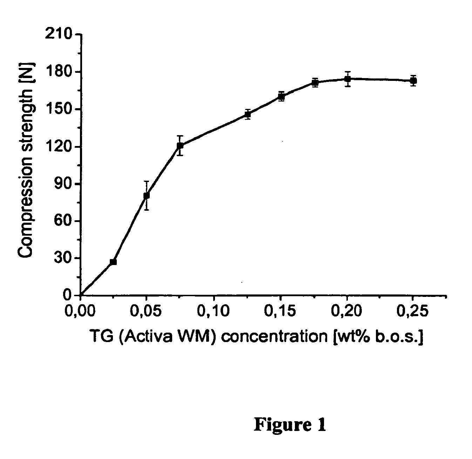

When no enzyme was used, the cylinder could be compressed without measurable force, whereas the compression strength could be increased up to 175 N upon adding 0.175 mass% (based on mass of sand) of enzyme (equivalent to 17.5 U/ g of protein). The addition of more enzyme gave no additional strength (see figure 1).

Figure 1 shows the compression strength of cylindrical test specimen in the wet state (diameter 50 mm, height: 50 mm), produced with sand, 1% sodium-caseinate and the enzyme transglutaminase (b.o.s. based on sand, wt% on a dry basis).

### Example 2

Protein extract was produced in the following manner: Emvital E 7 (pea protein, Emsland Stärke GmbH) was suspended in water and after sedimentation of the non soluble matter, the supernatant was centrifuged for further purification. The obtained clear protein solution was mixed with acetone (technical grade, 95 %) in a volume ratio of 1:2 (protein solution/ acetone) for precipitation of the protein. The suspension was again centrifuged to obtain the precipitated protein. This protein fraction was washed again with acetone and after sedimentation the overlaying acetone was removed. The remaining acetone was evaporated by storing the precipitated protein on a teflon foil at room temperature. After drying, the protein was ground to a fine powder and was ready to use.

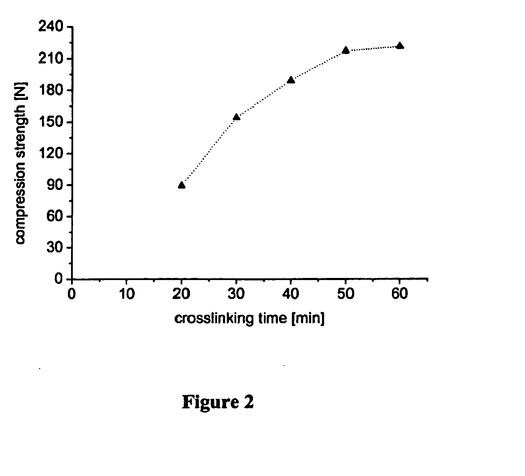

The dry protein powder was mixed with sand (1.3 % b.o.s.) in the dry state. Then, 5 % (b.o.s.) of buffer solution (Tris/HCl, pH 7, 20 mM) containing 0.25 % b.o.s. Activa WM was added to the sand protein mix. Compression specimens (cylindrical, 50 mm x 50 mm) were produced by weighing in 163 g of the sand mixture in a split specimen tube, compaction occurred with 3 ram blows. The covered moulds (to prevent evaporation of buffer) were then placed in a (preheated) drying oven at a temperature of 50 °C. In 10 min intervals (starting after 20 min) specimens were removed from the oven and immediately tested in the compression test. Testing speed was set to 50 mm/min. The crosslinking reaction lead to an increasing strength of the compression specimen (see Figure 2).

### Example 3

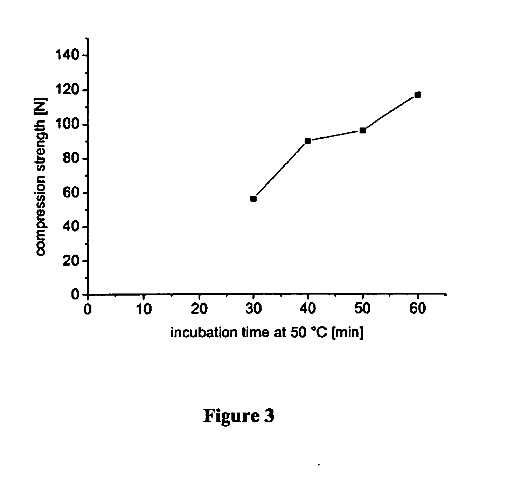

Gelatin was mixed with buffer and kept at room temperature for two hours to allow proper swelling of the gelatin. After that, the jelly mixture was heated up to 50 °C to form a homogenous gelatin solution. The warm solution was added to dry sand (H32) wherein 0.25 % (b.o.s.) Activa WM has been mixed before and a final concentration of 5 % buffer (b.o.s.) and 1 % gelatin (b.o.s.) was adjusted. The resulting activity of the enzyme was 25 U/g gelatin. Compression specimens were formed by weighing in 163 g of the mixture in split specimen tubes and compaction occurred with three ram blows. The covered tubes were then placed in a (preheated) drying oven at a temperature of 50 °C. In an interval of 10 minutes (starting after 30 min) the strength of the moist compression specimen was measured. There is an increasing strength of the specimen due to crosslinking of the gelatin (see Figure 3).

### Example 4

Crosslinking by a recombinant transglutaminase S2P (serine at position 2 exchanged against proline), expressed in and purified from E. coli.

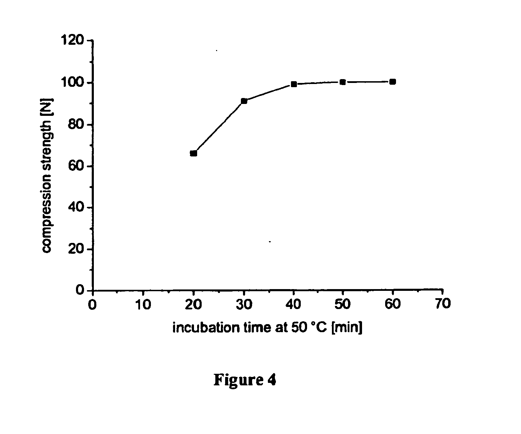

Pea protein extract (produced as described in Example 2) was mixed with sand in the dry state and buffer solution containing transglutaminase S2P with an activity of 66 U/g caseinate. After mixing, compression specimen were formed and tested as described in Example 1. The increase strength of the specimen due to the extracted pea protein is displayed in figure 4.

### Example 5, and comparative experiments A-D

The applicability of enzyme crosslinked proteins as binder for wood was tested by ABES (Automated Bonding Evaluation System, a device for testing adhesive bonds described in US 5,176,028).

Two pieces of beech veneer (173 mm times 20 mm each) were pressed together with an overlap of 5 mm times 20 mm with one drop of binder (0.15-0.20 mg) in between for 15 minutes at 50°C. Afterwards, the temperature was raised within 6 minutes towards 110°C and kept at this temperature for 5 minutes to remove water. Then, the pressure was released, the veneer pieces were pulled apart with the required force being measured. Table 1 shows the average value of 10 measurements.

**Table 1: Evaluation of binders with ABES**

| Exp. | Binder (12 wt.% in aqueous Tris/HCl buffer, 20 mM, pH 7,0) | Force [N] |
|---|---|---|
| A | Na-Caseinate | 470 |
| B | Enzyme* | 430 |
| 5 | Enzyme*: Na-Caseinate 1:4 | 575 |
| C | Enzyme,* cooked for 10 minutes : Na-Caseinate 1:4 | 376 |
| D | Enzyme,* cooked for 10 minutes | 112 |

| | | |
|---|---|---|
| * Enzyme = 1 wt.% of transglutammase and 99 wt.% of dextrin | | |

Comparative experiment A shows that proteins can be used as a binder. Experiment B demonstrates that even enzyme (which contains in fact of 99 wt.% of dextrin) can be used to bind wood. The experiment according to the invention, 5, shows that the force needed to break the bond is increased by more than 20% if enzyme and protein are combined. This is due to the chemical activity of the enzyme, as the strength cannot be increased anymore if the enzyme has been destroyed by boiling for 10 minutes (comparative experiments C and D).

### Example 6

Pieces of veneer were bonded together by the same binders and in the same way as described in the previous example, but were not pulled apart afterwards. Instead they were immersed in water at 20°C for 30 minutes. When either protein or enzyme was used as the sole binder, the binder dissolved and the veneer pieces separated. In the case where the enzyme and protein were combined in a 1:4 ratio, the veneer pieces were still attached to each other.

### Example 7

Pieces of veneer were bonded together by the same binders and in the same way as described in example 5, but were not pulled apart afterwards. Instead they were put in desiccators over water (100% humidity) at room temperature for 24 hours. With protein or enzyme used as the sole binder, the binder dissolved and the veneer pieces separated. In the case where the enzyme and protein were combined in a 1:4 ratio, the veneer pieces were still attached to each other.

Thus, the invention has been described by reference to certain embodiments discussed above. It will be recognized that these embodiments are susceptible to various modifications and alternative forms well known to those of skill in the art.

## Claims

1. Composite material comprising between 70 and 99.9 wt% (dry on dry) filler , wherein the filler is at least one of inorganic filler and wood and wherein the filler is in the form of fibres, particles, or sheets, and wherein the filler contains less than 20 wt% (relative to the filler) of proteinous material, and further comprising resin comprising proteinous material that contains proteins and/or polypeptides that are cross-linkable by enzymes, and enzymes suitable for said crosslinking.

2. Composite material according to claim 1, wherein the proteinous material used is from vegetable (including algal), animal, fungal, and/or microbial (bacteria, yeast) sources.

3. Composite material according to any one of claims 1-2, wherein the proteinous material is enriched by at least partially separating the proteins and/or polypeptides from other source substances .

4. Composite material according to any one of claims 1-3, wherein the proteinous material is hydrolysed to give polypeptides.

5. Composite material according to any one of claims 1-4, wherein the enzymes used for crosslinking are adapted to the envisioned reaction conditions.

6. Composite material according to any one of claims 1-5, wherein the crosslinking enzyme is a Transglutaminase (EC 2.3.2.13).

7. Composite material according to any one of claims 1-6, wherein the filler comprises a particle size of at least 0.2mm.

8. Composite product obtained by crosslinking the resin in a composite material according to any one of claims 1-7.

9. Composite products according to claim 8, wherein the composite product has a gradient in mechanical properties.

10. Use of the composite material of any one of claims 1-9 in the production of foundry moulds.

11. Use of the composite material of any one of claims 1-9 in the production of wood panels.

12. Process for the production of foundry moulds by shaping a composite material according to any one of claims 1-9, wherein the filler is sand and wherein the proteinous material that contains proteins and/or polypeptides is crosslinked by an enzymatic reaction.

13. Process for the production of wood panels by shaping a composite material according to any one of claims 1-9, wherein the filler is wood fibre, wood powder and/or veneer sheet and wherein the proteinous material that contains proteins and/or polypeptides is crosslinked by an enzymatic reaction.

14. Process for binding fillers with a resin comprising cross-linkable proteins or proteinous material that contains proteins and/or polypeptides, and cross-linking the proteinous material with enzymes, wherein the amount of filler is between 70 and 99.9 wt% (dry on dry) filler, wherein the filler comprises at least one of inorganic filler and wood and wherein the filler is in the form of fibres, particles, or sheets, and wherein the filler contains less than 20 wt% (relative to the filler) of proteinous material.

15. Two-component adhesives for fillers , wherein one component comprises a proteinous material that contains proteins and/or polypeptides, and the second component is an enzyme, capable of cross-linking the proteins and/or polypeptides in said proteinous material.

16. Coated particles for use in a process for making foundry moulds, the coated particles comprising inorganic particles having a size between 0.1mm and 5 mm and coated with a resin comprising proteinous material that contains proteins and/or polypeptides that are cross-linkable by enzymes suitable for said crosslinking.

## Patentansprüche

1. Verbundmaterial, das zwischen 70 und 99,9 Gew.% (trocken zu trocken) Füllstoff umfasst, wobei der Füllstoff wenigstens anorganischer Füllstoff und/oder Holz ist und wobei der Füllstoff in Form von Fasern, Teilchen oder Blättern vorliegt und wobei der Füllstoff weniger als 20 Gew.% (bezogen auf den Füllstoff) proteinartiges Material umfasst und das ferner ein Harz umfasst, welches proteinartiges Material umfasst, das Proteine und/oder Polypeptide enthält, die durch Enzyme quervernetzt werden können sowie zum Quervernetzen geeignete Enzyme.

2. Verbundmaterial nach Anspruch 1, wobei das verwendete proteinartige Material aus pflanzlichen, tierischen, pilzlichen und/oder mikrobiellen (Bakterien, Hefe) Quellen stammt.

3. Verbundmaterial nach einem der Ansprüche 1-2, wobei das proteinartige Material dadurch angereichert ist, dass die Proteine und/oder Polypeptide wenigstens teilweise von anderen Quellsubstanzen getrennt werden.

4. Verbundmaterial nach einem der Ansprüche 1-3, wobei das proteinartige Material zur Bildung von Polypeptiden hydrolysiert wird.

5. Verbundmaterial nach einem der Ansprüche 1-4, wobei die zum Quervernetzen eingesetzten Enzyme den voraussichtlichen Reaktionsbedingungen angepasst sind.

6. Verbundmaterial nach einem der Ansprüche 1-5, wobei das quervernetzende Enzym eine Transglutaminase (EC 2.3.2.13) ist.

7. Verbundmaterial nach einem der Ansprüche 1-6, wobei der Füllstoff eine Teilchengröße von wenigstens 0,2 mm umfasst.

8. Verbundwerkstoff, der durch Quervernetzen des Harzes in einem Verbundmaterial nach einem der Ansprüche 1-7 erhalten wird.

9. Verbundwerkstoffe nach Anspruch 8, wobei der Verbundwerkstoff einen Gradienten in den mechanischen Eigenschaften aufweist.

10. Verwendung des Verbundmaterials eines der Ansprüche 1-9 zur Herstellung einer Gießform.

11. Verwendung des Verbundmaterials eines der Ansprüche 1-9 zur Herstellung von Holzplatten.

12. Verfahren zur Herstellung von Gießformen durch Formen eines Verbundmaterials nach einem der Ansprüche 1-9, wobei der Füllstoff Sand ist und wobei das proteinartige Material, das Proteine und/oder Polypeptide enthält, durch eine enzymatische Reaktion quervernetzt wird.

13. Verfahren zur Herstellung von Holzplatten durch Formen eines Verbundmaterials nach einem der Ansprüche 1-9, wobei der Füllstoff Holzfasern, Holzpulver und/oder Furnierblatt ist und wobei das proteinartige Material, das Proteine und/oder Polypeptide enthält, durch eine enzymatische Reaktion quervernetzt wird.

14. Verfahren zum Verbinden von Füllstoffen mit einem Harz, das quervernetzbare Proteine oder proteinartiges Material umfasst, welches Proteine und/oder Polypeptide enthält sowie Quervernetzen des proteinartigen Materials mit Enzymen, wobei die Menge an Füllstoff zwischen 70 und 99,9 Gew.% (trocken zu trocken) Füllstoff beträgt, wobei der Füllstoff mindestens einen anorganischen Füllstoff und Holz umfasst und wobei der Füllstoff in Form von Fasern, Teilchen oder Blättern vorliegt und wobei der Füllstoff weniger als 20 Gew.% (bezogen auf den Füllstoff) proteinartiges Material enthält.

15. Zweikomponentenklebstoffe für Füllstoffe, wobei eine Komponente ein proteinartiges Material umfasst, das Proteine und/oder Polypeptide enthält und die zweite Komponente ein Enzym ist, das in der Lage ist, die Proteine und/oder Polypeptide in dem proteinartigen Material quer zu vernetzen.

16. Beschichtete Teilchen zur Verwendung in einem Verfahren zur Herstellung von Gießformen, wobei die beschichteten Teilchen anorganische Teilchen umfassen, die eine Größe zwischen 0,1 mm und 5 mm aufweisen und mit einem Harz beschichtet sind, das proteinartiges Material umfasst, welches Proteine und/oder Polypeptide enthält, die durch Enzyme quervernetzt werden können, die zum Quervernetzen geeignet sind.

## Revendications

1. Matériau composite comprenant entre 70 et 99.9 % en poids (sec) de charge (sèche), dans lequel la charge est au moins une charge minérale et du bois et dans lequel la charge est sous la forme de fibres, de particules, ou de feuilles, et dans lequel la charge contient moins de 20 % en poids (par rapport à la charge) de matériau protéiné, et comprenant en outre de la résine comprenant du matériau protéiné qui contient des protéines et/ou des polypeptides qui sont réticulables par des enzymes, et des enzymes appropriés pour ladite réticulation.

2. Matériau composite selon la revendication 1, dans lequel le matériau protéiné utilisé provient de sources végétale (y compris d'algues), animale, fongique, et/ou microbienne (bactéries, levure).

3. Matériau composite selon l'une quelconque des revendications 1 - 2, dans lequel le matériau protéiné est enrichi en séparant au moins partiellement les protéines et/ou les polypeptides d'autres substances sources.

4. Matériau composite selon l'une quelconque des revendications 1 à 3, dans lequel le matériau protéiné est hydrolysé pour donner des polypeptides.

5. Matériau composite selon l'une quelconque des revendications 1 à 4, dans lequel les enzymes utilisés pour la réticulation sont adaptés aux conditions réactionnelles envisagées.

6. Matériau composite selon l'une quelconque des revendications 1 à 5, dans lequel l'enzyme de réticulation est un Transglutaminase (EC 2.3.2.13).

7. Matériau composite selon l'une quelconque des revendications 1 à 6, dans lequel la charge comprend une taille de particule d'au moins 0,2 mm.

8. Produit composite obtenu par réticulation de la résine dans un matériau composite selon l'une quelconque ces revendications 1 à 7.

9. Produits composites selon la revendication 8, dans lesquels le produit composite a un gradient dans les propriétés mécaniques.

10. Utilisation du matériau composite selon l'une quelconque des revendications 1 à 9 dans la production de moulages de fonderie.

11. Utilisation du matériau composite selon l'une quelconque des revendications 1 à 9 dans la production de panneaux de bois.

12. Processus pour la production de moulages de fonderie par formation d'un matériau composite selon l'une quelconque des revendications 1 à 9, dans lequel la charge est du sable et dans lequel le matériau protéiné qui contient des protéines et/ou des polypeptides est réticulé par une réaction enzymatique.

13. Processus pour la production de panneaux de bois en formant un matériau composite selon l'une quelconque des revendications 1 à 9, dans lequel la charge est de la fibre de bois, de la poudre de bois et/ou du placage et dans lequel le matériau protéiné qui contient des protéines et/ou des polypeptides est réticulé par une réaction enzymatique.

14. Processus de liaison de charges avec une résine comprenant des protéines ou un matériau protéiné réticulable(s) qui contient des protéines et/ou des polypeptides, et de réticulation du matériau protéiné avec des enzymes, dans lequel la quantité de charge est entre 70 et 99,9 % en poids (sec) de charge (sèche), dans lequel la charge comprend au moins une charge minérale et du bois et dans lequel la charge est sous la forme de fibres, de particules, ou de feuilles, et dans lequel la charge contient moins de 20 % en poids (par rapport à la charge) de matériau protéiné.

15. Adhésifs à deux composants pour des charges, dans lesquels un composant comprend un matériau protéiné qui contient des protéines et/ou des polypeptides, et le deuxième composant est un enzyme, capable de réticuler les protéines et/ou les polypeptides dans ledit matériau protéiné.

16. Particules enduites à utiliser dans un processus de fabrication de moulages de fonderie, les particules enduites comprenant des particules minérales ayant une taille entre 0,1 mm et 5 mm et enduites d'une résine comprenant un matériau protéiné qui contient des protéines et/ou des polypeptides qui sont réticulables par des enzymes appropriés pour ladite réticulation.
